Europäisches Patentamt

European Patent Office

Office européen des brevets

(11) Publication number: 0 604 727 A1

# EUROPEAN PATENT APPLICATION

(21) Application number: 93117244.9

(22) Date of filing: 25.10.93

(51) Int. Cl.5: A61K 39/39, A61K 37/24

(30) Priority: 31.12.92 US 999066

(43) Date of publication of application:
06.07.94 Bulletin 94/27

(84) Designated Contracting States:
AT BE CH DE DK ES FR GB GR IE IT LI LU NL
PT SE

(71) Applicant: AMERICAN CYANAMID COMPANY
One Cyanamid Plaza
Wayne New Jersey 07470(US)

(72) Inventor: Daley, Michael Joseph
1564 Hummingbird Court
Yardley, Pennsylvania 19067(US)
Inventor: Frenchick, Patrick
6601 Rexford Drive
Lincoln, Nebraska 68506(US)
Inventor: Pillai, Subramonia P.
286 Vollmer Parkway
Rochester, New York 14623(US)

(74) Representative: Wächtershäuser, Günter, Dr.
Tal 29
D-80331 München (DE)

(54) Improved immunogenicity of a vaccine by incorporation of a cytokine within an immune-stimulating complex containing an antigen.

(57) This disclosure describes a vaccine comprising an effective immunizing amount of an immune-stimulating complex which contains an antigen and a cytokine. The composition may also contain conventional adjuvants, preservatives and a pharmaceutically acceptable carrier. The disclosure further describes a unique method for enhancing or accelerating the immunogenicity of weak antigens by parenterally administering to a mammal an effective immunizing amount of the aforesaid vaccine.

Rank Xerox (UK) Business Services
(3.10/3.09/3.3.4)

## BACKGROUND OF THE INVENTION

### Field of the Invention

The present invention relates to a new vaccine comprising a cytokine incorporated within an immune-stimulating complex in combination with a viral or bacterial antigen and the novel use of the vaccine to effectively improve the immunological response to the antigen.

### Description of the Related Art

Human and bovine respiratory syncytial virus (hereinafter referred to as "BRSV") is an important etiological agent in respiratory diseases of young children and calves. In 1968, it was disclosed that convalescent serum was capable of neutralizing respiratory syncytial virus (Doggett et al., A Study of an Inhibitor in Bovine Serum Active against Respiratory Syncytial Virus, *Archiv für die gesamte Virusforschung* 23:126-137 (1968)). More recently, a neutralizing epitope for RSV has been identified in the F1 fusion fragment of the fusion protein (Trudel et al., Identification of a Synthetic Peptide as Part of a Major Neutralization Epitope of Respiratory Syncytial Virus, J. Gen. Virol. 68:2273-2280 (1987)).

Interleukin-1 is a cytokine with multiple *in vivo* effects (Oppenheim et al., There is more than one interleukin-1, Immunol. Today 7:45-56 (1986); Dinarello, Interleukin-1, Rev. Infect. Dis. 6:652-695 (1984); Dinarello, Biology of Interleukin-1, FASEB. J. 2:108-115 (1988)). Previous studies have shown that r-BoIL-1$\beta$ is useful as an immunomodulator of bovine immune responses to viral infections (see Reddy et al., Adjuvanicity of recombinant bovine interleukin-1$\beta$: influence on immunity, infection and latency in bovine herpes virus-1 infection, Lymphokine Res. 9:295-300 (1990)). In these studies r-BoIL-1$\beta$-treatment of calves increased antibody production against bovine herpes virus-1 (BHV-1), bovine virus diarrhea (BVD) and parainfluenza-3 (PI-3) viruses, enhanced cytotoxic responses to virally infected MDBK cells, decreased viral shedding of BHV-1 after challenge and had lower recrudescence of BHV-1 following dexamethasone injections.

Immune-stimulating complexes (hereinafter referred to as "ISCOMs") are complexes of lipids with antigens and adjuvants. The use of ISCOMs as an effective means of presenting viral antigens, usually surface glycoproteins, has been described in the literature (Morein et al., Iscom, a novel structure for antigenic presentation of membrane proteins from enveloped viruses, Nature 308:457-460 (1984); see also U.S. Patent Nos. 4,900,549 and 4,744,983). ISCOMs have also been found to potentiate the immune response to a variety of vaccine antigens (Takahashi et al., Induction of CD8[+] cytotoxic T cells by immunization with purified HIV-1 envelope protein in ISCOMs, Nature 344:873-875 (1990); Erturk et al., Antibody responses and protection in mice immunized with herpes simplex virus type 1 antigen immune-stimulating complex preparations, J. Gen. Virol. 70:2149-2155 (1989); Trudel et al., Experimental polyvalent ISCOMs subunit vaccine induces antibodies that neutralize human and bovine respiratory syncytial virus, Vaccine 7:12-16 (1989)). Similarly, previous reports have suggested that recombinant bovine interleukin-1$\beta$ can potentiate the activity of antigens when administered subcutaneously in an aqueous solution (Reddy et al., Adjuvanicity of recombinant bovine interleukin-1$\beta$: influence on immunity, infection and latency in bovine herpes virus-1 infection, Lymphokine Res. 9:295-300 (1990)). It has not, however, been reported or suggested in the art that interleukin-1 can be effectively incorporated or if incorporated within a lipid vessicle, would be active and/or potentiate the activity of the antigen.

## OBJECTS OF THE INVENTION

It is therefore an important object of the present invention to provide a highly unique vaccine possessing significantly improved immunogenicity in mammals, particularly comprised of weak antigens.

Another object is to provide a novel method of preparing a vaccine to substantially improve the immunogenicity of the vaccine by incorporating a cytokine within an ISCOM containing an antigen.

A further object is to provide a new method of potentiating or accelerating the immunological activity of an antigen in a mammal.

Further purposes and objects of the present invention will appear as the specification proceeds.

The foregoing objects are accomplished by providing a vaccine in which a cytokine is formulated within ISCOMs in combination with a viral or bacterial antigen. The product of this invention produces highly improved immunological response to the antigen as compared to classical vaccines or to antigens incorporated into ISCOMs alone. The background of the invention and its departure from the art will be further described hereinbelow.

SUMMARY OF THE INVENTION

The present invention involves an improved vaccine which comprises an effective immunizing amount of an antigen and a cytokine incorporated within an immune-stimulating complex. The antigen can be any infectious agent which would be contemplated by those of ordinary skill in the art. The infectious agent, for example, can be viral, fungal or bacterial in nature. Also included as infectious agents are parasites, tumor antigens and antigens of other pathological diseases. The vaccine optionally contains conventional adjuvants and preservatives. A pharmaceutically acceptable carrier can be added to the vaccine at any convenient time. Additionally, the present invention concerns a novel method for enhancing the immunogenicity of weak or immunosuppressive antigens which comprises parenterally administering to a mammal an effective vaccinating amount of the aforedescribed composition.

BRIEF DESCRIPTION OF THE DRAWINGS

The background of the invention and its departure from the art will be further described hereinbelow with reference to the accompanying drawings, wherein:

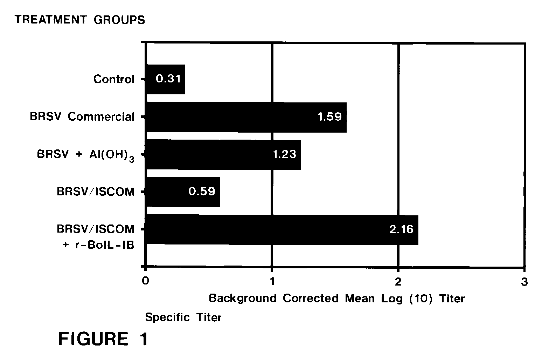

FIGURE 1 shows the potentiation of BRSV vaccines with ISCOMs in combination with interleukin-1$\beta$ through measurement of serum ELISA titers to bovine respiratory syncytial virus (BRSV). Calves are injected with the various vaccines on Day 0 and boosted on Day 28. Each of the five groups contain 3 calves randomly assigned based upon pre-titers to BRSV. The data represent the geometric mean titer four weeks post-immunization (secondary) taken from triplicate samples of each of the animals. The geometric mean titer is expressed as the mean $Log_{10}$ titer +/-S.D. The pre-immune titers have been subtracted from all groups.

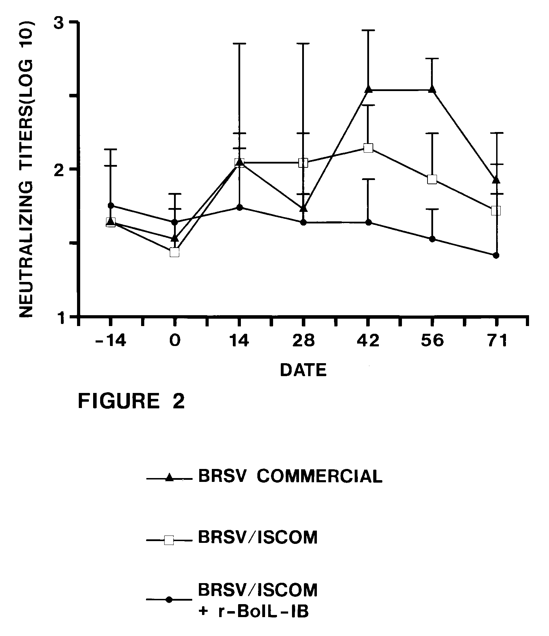

FIGURE 2 illustrates the neutralizing titers against BRSV through measurement of serum neutralization antibodies to BRSV. Calves are injected with the various vaccines on Day 0 and boosted on Day 28. The serum neutralizing titers for BRSV are measured at various times after immunization. Each point represents the mean +/- S.D. of three animals from triplicate determinations for BRSV commercial vaccine; BRSV/ISCOM; or BRSV/ISCOM + r-BoIL-1$\beta$.

DETAILED DESCRIPTION OF THE INVENTION

In accordance with the present invention, the novel vaccine comprises an effective immunizing amount of an immune-stimulating complex ("ISCOM") containing an antigen and a cytokine. Surprisingly, the direct incorporation of cytokines into ISCOMs significantly potentiates the immuno-modulatory ability and has wide applications for potentiating the biological activity of a variety of antigens.

A cytokine is incorporated within the ISCOM in different amounts but usually ranges from about 0.0001% to about 0.1% by weight. Typical cytokines useful in the vaccine of the present invention include, but are not limited to, bovine interleukin-1 (e.g., bovine interleukin-1$\alpha$, bovine interleukin-1$\beta$, bovine recombinant interleukin-1$\beta$),human interleukin-1, rat interleukin-1, interferons, tumor necrosis factor, transforming growth factor, colony stimulating factors and the like.

The ISCOMs may be prepared according to conventional procedures such as the processes described in U.S. Patent Nos. 4,900,549 and 4,744,983. Typically, the ISCOM consists of a complex formed between a lipid and an adjuvant or a mixture of adjuvants such as solubilizing agents, surfactants, foaming agents, emulsifying agents and the like. The lipid is usually a phospholipid, desirably phosphatidylcholine, and/or a sterol such as cholesterol and the like. In combination with the lipid, the ISCOM may contain a solubilizing agent for solubilizing the antigen. Examples of solubilizing agents would include, for example, detergents, preferably octylglucoside, and the like. The ISCOM may further contain a foaming agent or an emulsifying agent for saponification or emulsification of the composition. Examples of foaming agents would include, for example, a glycoside such as a saponin and the like. Quil A which is extracted from the bark of *Quillaja saponaria* Molina is a particularly preferred saponin for use in the vaccine of the present invention. Besides the above-described formulations, it is also anticipated that alternative ISCOM combinations which are well known in the art can be utilized in the invention with satisfactory results.

A wide variety of conventional parasitic, fungal, viral or bacterial antigens may be employed within the ISCOMs depending upon the desired results. Tumor antigens and antigens of other pathological diseases may similarly be employed within the ISCOMs. The antigen is incorporated with the cytokine within the ISCOM in varying amounts and typically ranges from about 0.000001% to about 1.0% by weight. Examples of typical viral antigens include, but are not limited to, respiratory syncytial virus, herpes simplex virus type 1 (HSV), bovine virus diarrhea (BVD), parainfluenza-3 virus (PI), etc. Typical bacterial antigens include, but are not limited to, *Pasteurella, Haemophilus, Salmonella, Staphylococcus, Streptococcus,* etc. Typical parasitic antigens include, but are not limited to, *Coccidia, Hemosporidia, Amoebida, Trypanosoma, Leishmania, Giardia, Histomonas,* etc. Typical fungal antigens include, but are not limited to, *Coccidioides, Histoplasma, Blastomyces, Aspergillus, Cryptococcus,* etc.

A pharmaceutically acceptable carrier is also present in the composition in varying amounts. The amount of carrier, of course, will be dependent upon the amounts selected for the other ingredients. The carrier can be added to the vaccine at any convenient time. In the case of a lyophilized vaccine, the carrier can, for example, be added immediately prior to administration. Alternatively, the final product can be manufactured with the carrier. Examples of appropriate nontoxic, inert carriers include, but are not limited to, sterile water, saline, buffers, etc.

Optionally, the composition may contain conventional adjuvants such as stabilizers, emulsifiers, aluminum hydroxide, bacterial cell walls, their derivatives, synthetics and the like. The adjuvants are added to the vaccine as processing aids in varying customary amounts depending on the adjuvant and the desired result. For purposes of this invention, these adjuvants are identified herein as "secondary" merely to contrast with the above-described adjuvants which are included in the immune-stimulating complex itself.

As needed, conventional preservatives can be added to the vaccine in effective amounts ranging from about 0.0001% to about 0.1% by weight. Depending on the preservative employed in the formulation, amounts below or above this range may be useful. Typical preservatives include, for example, potassium sorbate, sodium metabisulfite, methyl paraben, propyl paraben, thimerosal, etc.

Advantageously and surprisingly, by using the antigen and a cytokine in an ISCOM, a vaccine formulation is produced which is highly antigenic. The significantly improved immunogenicity or acceleration of the onset of action gives a profound departure from the state of the art.

The present invention further embraces the novel method for improving or accelerating the immunogenicity of weak or immunosuppressive antigens which comprises parenterally administering to a mammal an effective immunizing amount of the aforedescribed formulation. Preferably, the vaccine is administered subcutaneously but it can be administered intramuscularly, intradermally or orally, if desired. The dosage of the vaccine will be dependent upon the selected antigen and the route of administration. It is contemplated that a person of ordinary skill in the art can easily and readily titrate the dosage for an immunogenic response for each antigen and method of administration.

In the practice of this invention, the following examples demonstrate that the incorporation of a cytokine such as r-BoIL-1$\beta$ into the BRSV/ISCOMs greatly enhances the immunogenicity of the BRSV. While the BRSV/ISCOMs are only marginally effective as a vaccine alone, the combination with r-BoIL-1$\beta$ unexpectedly shows a thirty-fold enhancement of the humoral response. This enhancement is also represented in the serum neutralizing titers as well. As shown below, the BRSV/ISCOM + r-BoIL-1$\beta$ group yields the highest specific titer and net geometric mean serum neutralizing titers. It is additionally observed that calves administered BRSV/ISCOM with r-BoIL-1$\beta$ exhibit significant titers sooner and are significantly higher than animals administered BRSV/ISCOM alone. This group also tends to yield higher titers than the commercial BRSV vaccine.

A further understanding of the present invention can be obtained from the following examples. However, the examples are set forth only for the illustration of certain aspects of the invention and are not to be construed as limitations thereon. Unless otherwise expressed, all parts are by weight.

EXAMPLE 1

Lymphocyte Proliferative Response to RSV Antigen

Fifteen Holstein calves, 4-6 months of age, are randomized into five groups of three, based upon their pre-bleed titers to BRSV. The five groups are: 1) Control which is injected with phosphated buffered saline (PBS); 2) vaccinates with a commercial BRSV vaccine; 3) vaccinates with a BRSV vaccine containing aluminum hydroxide; 4) vaccinates given ISCOMs/BRSV and 5) vaccinates given ISCOMs/BRSV encapsulated in the presence of recombinant bovine interleukin-1$\beta$ ("r-BoIL-1$\beta$"). All calves are vaccinated on day 0 and 28 of the study with the respective vaccines. On days -83, -54, -14, -7, 0, 14, 28, 42, 56 and 71, all calves are bled for serum titers to BRSV.

The commercial vaccine is purchased from Bioceutics (St. Joseph, MO). To make the vaccine designated BRSV + Al(OH)$_3$, a BRSV culture with a titer of $10^5$ pfu/mL is prepared and concentrated 4-fold. The concentrated BRSV material is UV inactivated and absorbed onto aluminum hydroxide. This BRSV is not assayed for viable virus following irradiation because previous work has established that approximately log 5.6 to log 6.2 is required for a protective dose of vaccine.

Immune Stimulating Complexes with BRSV (ISCOM/BRSV) are prepared by taking a concentrated BRSV culture (equivalent dose for 20 calves) and mixing this with 5% octylglucoside detergent with subsequent sonication. To this mixture is added 2 mg Quil A, 500 $\mu$g phosphatidylcholine and 500 $\mu$g of cholesterol. The total mixture is sonicated and then dialyzed for 72 hours. Similarly, ISCOM/BRSV + r-BoIL-1$\beta$ are prepared by adding 700 $\mu$g of r-BoIL-1$\beta$ (35 $\mu$g/vaccine dose) to the Quil A plus lipid mixture as described above. Analysis of the ISCOMs demonstrates that approximately 30% to 50% of the BRSV F and G proteins are incorporated within the complex. In addition, 20% to 40% of the r-BoIL-1$\beta$ is also shown to be associated with the ISCOMs.

None of the vaccinated animals enhance their antigen specific lymphocyte proliferation to either the F protein from human A2 strain of RSV or to UV inactivated BRSV over non-vaccinated animals or their own pre-vaccinate levels. The only animals that tend to respond to antigen stimulation are responsive prior to vaccination and tend to remain so throughout the study. The addition of r-BoIL-1$\beta$ does not influence any of these responses. None of the animals which are originally negative for antigen specific proliferation are converted to positive after vaccination.

The lymphocyte proliferative responses to the RSV antigens following immunization with BRSV are set forth in Table 1 below. The results illustrate that a greater percentage of the animals immunized with an ISCOM/RSV + IL-1$\beta$ preparation tend to demonstrate a T cell proliferative response (cellular immunity) at most time points as compared to the RSV + ISCOM animals.

Table 1.  Lymphocyte Proliferative Responses to RSV Antigens Following Immunization with BRSV

| Experimental Group | Animal Nos. | Day -14 | | Day 0[3] | | Day 14 | | Day 28[3] | | Day 42 | | Day 71 | |
|---|---|---|---|---|---|---|---|---|---|---|---|---|---|
| | | F[1] | BRSV[2] | F | BRSV | F | BRSV | F | BRSV | F | BRSV | F | BRSV |
| BRSV + Al(OH)₃ | 90 | - | + | + | - | +/- | + | + | + | + | +/- | +/- | + |
| | 97 | - | - | + | - | - | - | - | - | - | - | - | - |
| | K100 | - | - | + | - | +/- | - | + | - | +/- | +/- | - | - |
| BRSV Commercial | 94 | +/- | + | + | - | + | + | + | + | + | +/- | + | + |
| | K105 | +/- | +/- | + | - | + | + | + | - | + | - | - | - |
| | F108 | - | +/- | + | - | - | - | +/- | - | - | - | - | - |
| BRSV ISCOMs | 99 | +/- | + | + | +/- | + | + | + | + | + | + | + | + |
| | M113 | - | - | - | - | + | - | + | - | - | - | - | - |
| | K119 | - | - | +/- | - | - | - | +/- | - | - | - | - | - |
| BRSV ISCOMs+ IL-1β | 103 | - | - | + | - | + | + | + | + | + | +/- | + | +/- |
| | M111 | + | + | +/- | +/- | +/- | +/- | + | - | + | + | - | - |
| | L122 | - | - | + | + | + | - | +/- | - | - | - | - | - |
| Control | 91 | + | + | + | +/- | + | + | + | + | + | +/- | + | - |
| | 105 | + | + | + | - | + | + | + | + | + | +/- | nd | nd |
| | K120 | - | - | + | - | +/- | - | - | - | +/- | - | - | - |

1 Purified Human RSV subtype A and F proteins are used as antigens in LST assays at two dilutions.
2 Bovine RSV grown in MDBK cells is UV inactivated and after cell debris is removed, bovine RSV is used as LST antigen at two dilutions. Similarly

EXAMPLE 2

Serum Titers to RSV

The calves and procedures of Example 1 are used to determine the serum titers to BRSV. Total antibody titers and neutralizing antibody titers to BRSV are assayed using conventional materials and techniques.

The net specific serum ELISA titer to each of the vaccinate groups and controls are shown in Figure 1. The BRSV/ISCOM + r-BoIL-1$\beta$ group yields the highest specific titer after boosting, followed by the commercial BRSV vaccine. The net specific titer for the BRSV/ISCOM + r-BoIL-1$\beta$ group ($Log_{10}$ = 2.3) is almost thirty-fold greater than the BRSV/ISCOM group ($Log_{10}$ = 0.8), and a little more than two-fold greater than the commercial vaccine ($Log_{10}$ = 1.9). The titer also tends to remain higher in the BRSV/ISCOM + r-BoIL-1$\beta$ group for up to 4 weeks after boosting.

The serum neutralizing titers to BRSV for the commercial vaccine, BRSV/ISCOM group, and BRSV/ISCOM + r-BoIL-1$\beta$ group are shown in Figure 2. The serum neutralizing titer to BRSV shows a significant titer 2 weeks after primary immunization for both the commercial BRSV vaccine and the BRSV/ISCOM + r-BoIL-1$\beta$ group. In contrast, there is no significant titer over background for the BRSV/ISCOM group. Upon boosting with vaccine, the BRSV/ISCOM + r-BoIL-1$\beta$ group tends to have higher geometric mean serum neutralizing titers to BRSV (six- to eight-fold greater). Thus, the BRSV/ISCOM + r-BoIL-1$\beta$ group yields the highest specific antibody titer to the F protein after boosting, followed by the commercial BRSV vaccine.

Although some enhanced antibody titer is observed in the RSV/ISCOM group, these titers tend to be significantly lower than the commercial vaccine. In contrast, the RSV/ISCOM + IL-1$\beta$ group yields the highest specific titer and net geometric mean serum neutralizing titers. It is shown that calves administered RSV/ISCOM with IL-1$\beta$ demonstrate significant titers sooner and are significantly higher than animals administered RSV/ISCOM alone. This group also tends to yield higher titers than the commercial BRSV vaccine.

In the foregoing, there has been provided a detailed description of particular embodiments of the present invention for the purpose of illustration and not limitation. It is to be understood that all other modifications, ramifications and equivalents obvious to those having skill in the art based on this disclosure are intended to be included within the scope of the invention as claimed.

**Claims**

1. A vaccine which comprises an effective immunizing amount of an immune-stimulating complex containing an antigen and a cytokine.

2. The vaccine according to Claim 1, wherein the cytokine is interleukin-1, interferon, tumor necrosis factor, transforming growth factor or colony stimulating factor.

3. The vaccine according to Claim 2, wherein the cytokine is recombinant bovine interleukin-1.

4. The vaccine according to Claim 3, wherein the immune-stimulating complex contains an antigen selected from the group consisting of respiratory syncytial virus, herpes simplex virus type 1, bovine virus diarrhea, parainfluenza-3, *Pasteurella*, *Haemophilus, Salmonella*, *Staphylococcus, Streptococcus*, *Coccidia*, *Hemosporidia*, *Amoebida*, *Trypanosoma*, *Leishmania*, *Giardia*, *Histomonas*, *Coccidioides*, *Histoplasma*, *Blastomyces*, *Aspergillus* and *Cryptococcus.*

5. The vaccine according to Claim 4, wherein the immune-stimulating complex comprises at least one lipid, a solubilizing agent and a foaming agent.

6. The vaccine according to Claim 5, wherein a first lipid is a phospholipid, a second lipid is a sterol, the solubilizing agent is a detergent, and the foaming agent is a glycoside.

7. The vaccine according to Claim 6, wherein the phospholipid is phosphatidylcholine, the sterol is cholesterol, the detergent is octylglucoside, and the glycoside is Quil A.

8. The vaccine according to Claim 7, which comprises bovine respiratory syncytial virus in combination with phosphatidylcholine, cholesterol, octylglucoside and Quil A.

9. The vaccine according to Claim 1, further comprising a preservative.

10. The vaccine according to Claim 1, further comprising a secondary adjuvant or a pharmaceutically acceptable carrier.

**11.** A method for enhancing or accelerating the immunogenicity of a weak or immunosuppressive antigen which comprises parenterally administering to a mammal an effective immunizing amount of a vaccine comprising an immune-stimulating complex containing a weak or immunosuppressive antigen and a cytokine.

**12.** The method according to Claim 11, which comprises administering the vaccine which contains interleukin-1, interferon, tumor necrosis factor, transforming growth factor or colony stimulating factor as the cytokine.

**13.** The method according to Claim 12, which comprises administering the vaccine which contains recombinant bovine interleukin-1 as the cytokine.

**14.** The method according to Claim 13, which comprises administering the vaccine which contains the antigen selected from the group consisting of respiratory syncytial virus, herpes simplex virus type 1, bovine virus diarrhea, parainfluenza-3, *Pasteurella*, *Haemophilus*, *Salmonella*, *Staphylococcus*, *Streptococcus, Coccidia, Hemosporidia, Amoebida, Trypanosoma, Leishmania, Giardia, Histomonas, Coccidioides, Histoplasma, Blastomyces, Aspergillus* and *Cryptococcus*.

**15.** The method according to Claim 14, which comprises administering the vaccine which contains the immune-stimulating complex in which said complex comprises at least one lipid, a solubilizing agent and a foaming agent.

**16.** The method according to Claim 15, which comprises administering the vaccine which contains the immune-stimulating complex in which said complex comprises a phospholipid, a sterol, a detergent and a glycoside.

**17.** The method according to Claim 16, which comprises administering the vaccine which contains the immune-stimulating complex in which said complex comprises phosphatidylcholine, cholesterol, octylglucoside and Quil A.

**18.** The method according to Claim 17, which comprises administering the vaccine which contains bovine respiratory syncytial virus.

**19.** The method according to Claim 18, which comprises administering the vaccine subcutaneously.

TREATMENT GROUPS

FIGURE 1

FIGURE 2

—▲— BRSV COMMERCIAL

—□— BRSV/ISCOM

—●— BRSV/ISCOM
+ r-BoIL-IB

## DOCUMENTS CONSIDERED TO BE RELEVANT

EP 93117244.9

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 113, no. 15, 8 October, 1990, Columbus, Ohio, USA C. FOSSUM et al. "Effect of iscoms and their adjuvant moiety (matrix) on the initial proliferation and IL-2 responses: comparsion of spleen cells from mice inoculated with iscoms and/or matrix" page 473, abstract-no. 130 273d & Cell. Immunol. 1990, 129(2), 414-25 | 1-14 | A 61 K 39/39<br>A 61 K 37/24 |
| A | EP - A - 0 415 794 (COOPERS ANIMAL HEALTH LIMITED) * Abstract; claims * | 1-14 | |
| A | EP - A - 0 180 564 (MOREIN) * Abstract; claims * | 1-14 | TECHNICAL FIELDS SEARCHED (Int. Cl.5)<br><br>A 61 K |
| A | EP - A - 0 109 942 (MOREIN) * Abstract; claims * | 1-14 | |

The present search report has been drawn up for all claims

| Place of search VIENNA | Date of completion of the search 08-04-1994 | Examiner SCHNASS |
|---|---|---|